# EUROPEAN PATENT APPLICATION

(11) **EP 3 981 470 A1**
(43) Date of publication of application: **13.04.2022**
(21) Application number: 20817960.6
(22) Date of filing: 04.06.2020
(51) Int. Cl.: A61P 19/06, A61P 43/00, A61K 9/48, A61K 31/519

(54) **ENTERIC-COATED PREPARATION COMPRISING XANTHINE OXIDASE INHIBITOR**

(30) Priority: 04.06.2019 JP 2019104533
(71) Applicant: Nippon Chemiphar Co., Ltd., Tokyo 101-0032 (JP)
(72) Inventor: HIRANO Masuharu, Misato-shi, Saitama 341-0005 (JP); OHTA Takashi, Misato-shi, Saitama 341-0005 (JP)
(74) Representative: V.O.
(86) International application number: PCT/JP2020/022036
(87) International publication number: WO 2020/246526

(57) **Abstract**

The present invention provides an enteric-coated preparation, including: a compound represented by General 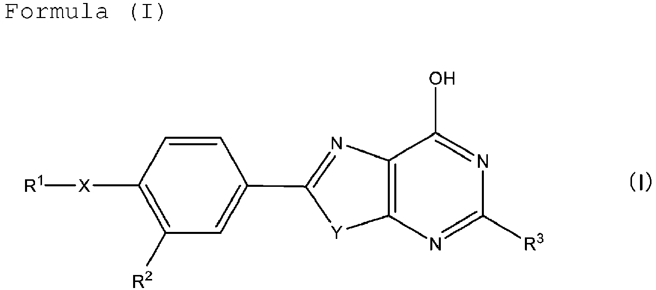 wherein R¹ is an unsubstituted phenyl group or a phenyl group substituted with a substituent, the substituent being at least one group selected from the group consisting of an alkyl group having 1 to 8 carbon atoms, an alkyl group having 1 to 8 carbon atoms substituted with a halogen atom, an alkoxy group having 1 to 8 carbon atoms, an alkoxycarbonyl group having 2 to 8 carbon atoms, a formyl group, a carboxyl group, a halogen atom, a phenyl group and a phenoxy group, R² is a cyano group or a nitro group, R³ is a hydrogen atom or a hydroxyl group, X is an oxygen atom or -S(O)ₙ-, n is an integer of 0 to 2, and Y is an oxygen atom or a sulfur atom, or a pharmaceutically acceptable salt thereof.

## Description

### TECHNICAL FIELD

The present invention relates to an enteric-coated preparation containing a compound having high xanthine oxidase inhibitory activity or a pharmaceutically acceptable salt thereof.

Priority is claimed on Japanese Patent Application No. 2019-104533, filed June 4, 2019, the content of which is incorporated herein by reference.

### BACKGROUND ART

Hyperuricemia causes gout, kidney failure, and the like, and is considered to be a risk factor for coronary artery disease. It has been pointed out that hyperuricemia has a close relationship with the onset of lifestyle-related diseases such as hypertension. Therefore, the treatment of hyperuricemia includes not only treatment of gout but also prevention of various lifestyle-related diseases associated with aging.

Currently, xanthine oxidase inhibitors such as allopurinol and febuxostat are mainly used for the treatment of hyperuricemia.

Known xanthine oxidase inhibitors such as allopurinol and febuxostat have been developed as solid preparations, more specifically, gastric-soluble tablets (for example, Non Patent Literature 1 and Non Patent Literature 2). Solid preparations have merits such as being suitable for mass production or being convenient to carry from the patient's point of view, and are the most widely applied dosage form in pharmaceuticals. These drugs exert sufficient efficacy when tablets rapidly disintegrate in the stomach and drugs are absorbed.

In addition, a compound described in PCT International Publication No. WO 2005/121153 pamphlet (Patent Literature 1) has been reported as a compound having a similar mechanism.

Patent Literature 1 discloses a comparative experiment on the plasma uric acid level suppression rate between the invention compound and the allopurinol and febuxostat in the pharmacological experiment 2 of Example 51. In this experiment, a gastric-soluble preparation is intended, and drug suspension administration in which methyl cellulose is used is performed. As a result, the invention compound exhibits an excellent uric acid level lowering effect.

The inventors made a study on a compound having a high xanthine oxidase inhibitory activity disclosed in

Patent Literature 1 and found that there was still room for improvement in in vivo absorbability when this compound was orally administered to a living body.

### CITATION LIST

### PATENT DOCUMENTS

Patent Literature 1: WO 2005/121153

### NON-PATENT DOCUMENTS

Non Patent Literature 1: Febuxostat US Package Insert February 13, 2009
Non Patent Literature 2: Zyloric US Package Insert Review July 26, 2002

### SUMMARY OF THE INVENTION

### PROBLEMS TO BE SOLVED BY THE INVENTION

Objects of the present invention are to improve in vivo absorbability of the compound disclosed in Patent Literature 1 or a pharmaceutically acceptable salt thereof, and provide a pharmaceutical composition containing them as an active ingredient.

### MEANS TO SOLVE THE PROBLEMS

The inventors conducted various studies regarding improvement in absorbability of the compound disclosed in Patent Literature 1 or a pharmaceutically acceptable salt thereof, and as a result found that an enteric-coated preparation of the compound disclosed in Patent Literature 1 or a pharmaceutically acceptable salt thereof can improve in vivo absorbability, thus completing the present invention.

Specifically, the present invention provides the following aspects.
[1] An enteric-coated preparation, including: a compound represented by General Formula (I): [0009] 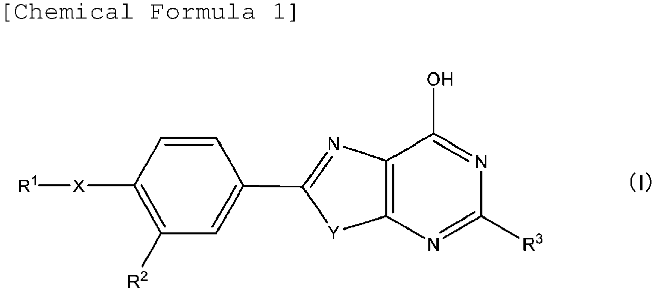 wherein R¹ is an unsubstituted phenyl group or a phenyl group substituted with a substituent, the substituent being at least one group selected from the group consisting of an alkyl group having 1 to 8 carbon atoms, an alkyl group having 1 to 8 carbon atoms substituted with a halogen atom, an alkoxy group having 1 to 8 carbon atoms, an alkoxycarbonyl group having 2 to 8 carbon atoms, a formyl group, a carboxyl group, a halogen atom, a phenyl group and a phenoxy group, R² is a cyano group or a nitro group, R³ is a hydrogen atom or a hydroxyl group, X is an oxygen atom or -S(O)ₙ-, n is an integer of 0 to 2, and Y is an oxygen atom or a sulfur atom, or a pharmaceutically acceptable salt thereof.
[2] The enteric-coated preparation according to [1], wherein R¹ is an unsubstituted phenyl group or a phenyl group substituted with a halogen atom.
[3] The enteric-coated preparation according to [1] or [2], wherein X is an oxygen atom.
[4] The enteric-coated preparation according to any one of [1] to [3], wherein Y is a sulfur atom;
[5] The enteric-coated preparation according to any one of [1] to [4], wherein the compound or a pharmaceutically acceptable salt thereof according to any one of [1] to [4] includes an amorphous form thereof, and a content of the amorphous form is 80 weight% or more based on a total weight of the compound or a pharmaceutically acceptable salt thereof according to any one of [1] to [4]; or
[6] The enteric-coated preparation according to any one of [1] to [5], which is a hard capsule.

### EFFECTS OF THE INVENTION

The enteric-coated preparation containing a xanthine oxidase inhibitor of the present invention is useful as a therapeutic agent for hyperuricemia and the like because it exhibits high in vivo absorbability.

### BRIEF DESCRIPTION OF THE DRAWINGS

Fig. 1 is a diagram showing a particle size distribution of Compound 14 by an amorphous wet laser diffraction method.
Fig. 2 is a diagram showing plasma drug concentration-time curves when the capsule of Example 1 and the capsule of Comparative Example 1 were administered to dogs.
Fig. 3 is a diagram showing a crystalline powder X-ray diffraction pattern of Compound 14.

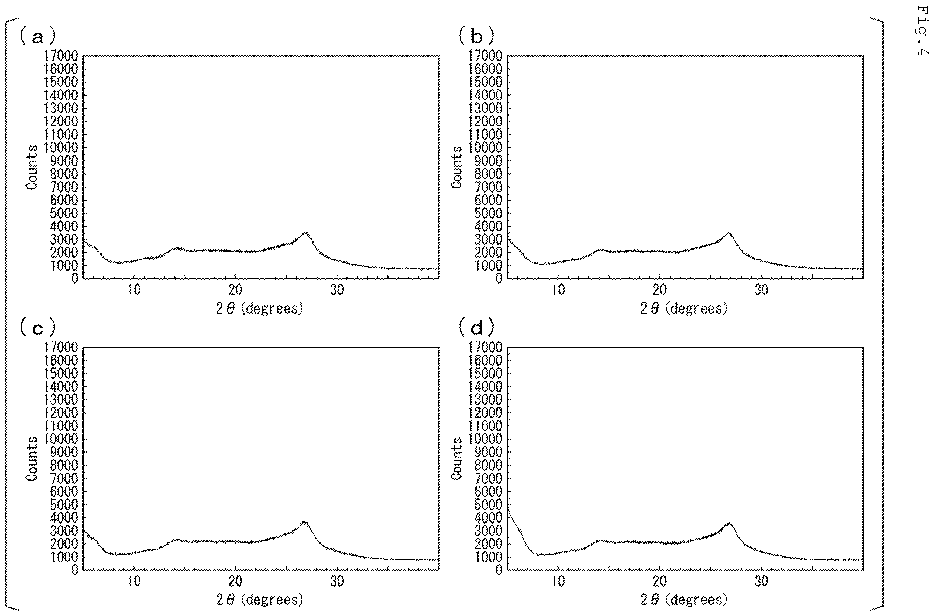

Fig. 4 is a plurality of diagrams showing amorphous powder X-ray diffraction patterns of Compound 14; and (a) is a diagram showing a powder X-ray diffraction pattern before storage, (b) is a diagram showing a powder X-ray diffraction pattern after storage under light-shielded/airtight and room temperature conditions for one week, (c) is a diagram showing a powder X-ray diffraction pattern after storage under light-shielded/airtight and room temperature conditions for two weeks, and (d) is a diagram showing a powder X-ray diffraction pattern after storage under light-shielded/airtight and room temperature conditions for four weeks.

### EMBODIMENTS FOR CARRYING OUT THE INVENTION

The present invention will be described below in further detail. The enteric-coated preparation of the present invention includes a compound represented by General Formula (I) or a pharmaceutically acceptable salt thereof as an active ingredient.

In a compound represented by General Formula (I), R¹ is an unsubstituted phenyl group or a phenyl group substituted with a substituent.

Examples of "an alkyl group having 1 to 8 carbon atoms" as a substituent in the phenyl group represented by R¹ include a methyl group, an ethyl group, a propyl group, an isopropyl group, a butyl group, an isobutyl group, a tert-butyl group, a pentyl group, and a hexyl group, and a methyl group or an ethyl group is preferable.

Examples of "an alkyl group having 1 to 8 carbon atoms substituted with a halogen atom" as a substituent in the phenyl group represented by R¹ include a fluoromethyl group, a trifluoromethyl group, a 1,1-difluoroethyl group, and a pentafluoroethyl group, and a fluoromethyl group or a trifluoromethyl group is preferable.

Examples of "an alkoxy group having 1 to 8 carbon atoms" as a substituent in the phenyl group represented by R¹ include a methoxy group, an ethoxy group, a propoxy group, an isopropoxy group, a butoxy group, an isobutoxy group, and a tert-butoxy group, and a methoxy group is preferable.

Examples of "an alkoxycarbonyl group having 2 to 8 carbon atoms" as a substituent in the phenyl group represented by R¹ include a methoxycarbonyl group, an ethoxycarbonyl group, a propoxycarbonyl group, a butoxycarbonyl group, and a tert-butoxycarbonyl group, and a methoxycarbonyl group or an ethoxycarbonyl group is preferable.

Examples of a "halogen atom" as a substituent in the phenyl group represented by R¹ include a fluorine atom, a chlorine atom, a bromine atom, and an iodine atom, and a fluorine atom or a chlorine atom is preferable.

Regarding R¹, an unsubstituted phenyl group is preferable.

In the compound represented by General Formula (I), R² is a cyano group or a nitro group, and a cyano group is preferable.

In the compound represented by General Formula (I), R³ is a hydrogen atom or a hydroxy group, and a hydrogen atom is preferable.

In the compound represented by General Formula (I), X is an oxygen atom or -S(O)ₙ-, and an oxygen atom is preferable.

In the compound represented by General Formula (I), Y is an oxygen atom or a sulfur atom, and a sulfur atom is preferable.

Examples of the pharmaceutically acceptable salt of the compound represented by General Formula (I) include an alkali metal salt such as a sodium salt, a potassium salt, and a lithium salt, and a potassium salt is preferable.

The compound of General Formula (I) contained in the enteric-coated preparation according to one embodiment of the present invention can be obtained, for example, by the synthetic method described in Patent Literature 1 or International Application PCT/JP2019/17439.

Regarding the compound of General Formula (I) contained in the enteric-coated preparation of the present invention, compounds in Table 1 may be exemplified as preferable compounds. In the table, Me is a methyl group.

**[Table 1]**

| | Structural formula | Compound name |
|---|---|---|
| Compound 1 | 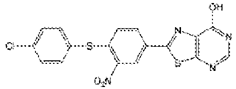 | 2-[4-(4-Chlorophenylthio)-3-nitrophenyl]-7-hydroxythiazolo[5,4-d]pyrimidine |
| Compound 2 | 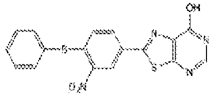 | 7-Hydroxy-2-(3-nitro-4-phenylthiophenyl)thiazolo[5,4-d]pyrimidine |
| Compound 3 | 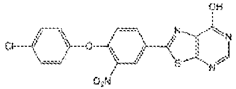 | 2-[4-(4-Chlorophenoxy)-3-nitrophenyl]-7-hydroxythiazolo[5,4-d]pyrimidine |
| Compound 4 | 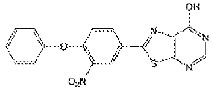 | 7-Hydroxy-2-(3-nitro-4-phenoxyphenyl) thiazolo[5,4-d]pyrimidine |
| Compound 5 | 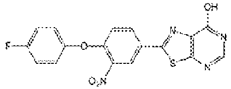 | 2-[4-(4-Fluorophenoxy)-3-nitrophenyl]-7-hydroxythiazolo[5,4-d]pyrimidine |
| Compound 6 | 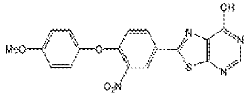 | 2-[4-(4-Methoxyphenoxy)-3-nitrophenyl]-7-hydroxythiazolo[5,4-d]pyrimidine |
| Compound 7 | 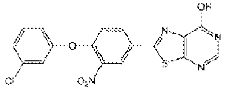 | 2-[4-(3-Chlorophenoxy)-3-nitrophenyl]-7-hydroxythiazolo[5,4-d]pyrimidine |
| Compound 8 | 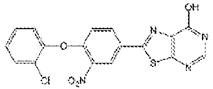 | 2-[4-(2-Chlorophenoxy)-3-nitrophenyl]-7-hydroxythiazolo[5,4-d]pyrimidine |
| Compound 9 | 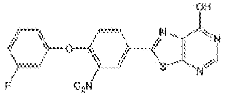 | 2-[4-(3-Fluorophenoxy)-3-nitrophenyl]-7-hydroxythiazolo[5,4-d]pyrimidine |
| Compound 10 | 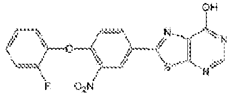 | 2-[4-(2-Fluorophenoxy)-3-nitrophenyl]-7-hydroxythiazolo[5,4-d]pyrimidine |
| Compound 11 | 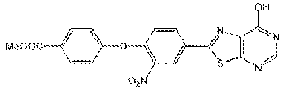 | 7-Hydroxy-2-[4-(4-methoxycarbonylphenoxy)-3-nitrophenyl]thiazolo[5,4-d]pyrimidine |
| Compound 12 | 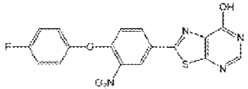 | 2-[4-(4-Fluorophenoxy)-3-nitrophenyl]-7-hydroxyoxazolo[5,4-d]pyrimidine |
| Compound 13 | 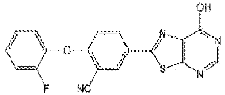 | 2-[3-Cyano-4-(2-fluorophenoxy)phenyl]-7-hydroxythiazolo[5,4-d]pyrimidine |
| Compound 14 | 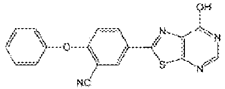 | 2-(3-Cyano-4-phenoxyphenyl)-7-hydroxythiazolo[5,4-d]pyrimidine |
| Compound 15 | 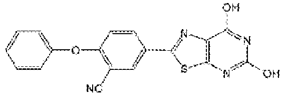 | 5-(5, 7-Dihydroxythiazolo[5, 4-d]pyrimidine-2-yl)-2-phenoxybenzonitrile |

Pharmaceutically acceptable salts may be formed from Compound 1 to Compound 15. Among these, Compound 3 to Compound 5, Compound 8 to Compound 10, Compound 13 to Compound 14, or pharmaceutically acceptable salts of these compounds are preferable.

In the "enteric-coated preparation" of the present invention, it is preferable that a part or all of the compound represented by General Formula (I) or a pharmaceutically acceptable salt thereof be amorphous. Here, the term "amorphous" means that a substance has a form having a short-distance order between atoms or molecules of the compound represented by General Formula (I) or a pharmaceutically acceptable salt thereof and having no long-distance order such as in crystals. In the present invention, an amorphous state can be identified according to a halo peak shown in X-ray diffraction.

In the present invention, based on a total weight of the compound represented by General Formula (I) or a pharmaceutically acceptable salt thereof, an amorphous content is preferably 50 weight% or more, more preferably 80 weight% or more, more preferably 90 weight% or more, and still more preferably 95 weight% or more, and an amorphous content may be 100 weight%. A pharmaceutically acceptable salt of the compound represented by General Formula (I) may be crystalline, and in this case, based on a total weight, an amorphous content may be less than 50 weight%, 40 weight% or less, 30 weight% or less, 20 weight% or less, 10 weight%, or 0 weight%. The amorphous content can be obtained by an X-ray diffraction method. For the amorphous content, the remaining content is a crystalline content. That is, in the description of each content, the total of the amorphous content and the crystalline content is 100 weight%.

An amorphous form of the compound represented by General Formula (I) or a pharmaceutically acceptable salt thereof can be produced, for example, by subjecting the compound represented by General Formula (I) or a pharmaceutically acceptable salt thereof to a spray drying method. More specifically, the compound represented by General Formula (I) or a pharmaceutically acceptable salt thereof and optionally a pharmaceutically acceptable additive may be added to a solvent described below to prepare a solution or suspension, the solution or suspension may be formed into a fine mist by centrifugal spraying using a rotating disk or pressure spraying using a pressure nozzle, and this may be sprayed into a drying medium (for example, heated air or nitrogen gas) to obtain an amorphous form of the compound represented by General Formula (I) or a pharmaceutically acceptable salt thereof as an amorphous powdery dried product. In the spray drying method, the temperature of the drying medium is, for example, 50 to 120 °C, and preferably 50 to 100 °C. The drying medium may be caused to flow in a certain direction, and can be caused to flow as an air flow, for example, at 0.1 to 0.6 m³/min.

Solvents used in the spray drying method include alcohols such as alcohols having 1 to 6 carbon atoms such as methanol, ethanol, 1-propanol, 2-propanol, and tert-butyl alcohol, and ethers such as tetrahydrofuran (THF), acetonitrile, and water, and these solvents can be used alone or as a mixed solvent of two or more types. Among these, ethanol, tetrahydrofuran, and a mixed solvent of these solvents and water are preferable.

Examples of another method for producing an amorphous form of the compound represented by General Formula (I) or a pharmaceutically acceptable salt thereof include a freeze-drying method. More specifically, an amorphous form of the compound represented by General Formula (I) or a pharmaceutically acceptable salt thereof can also be produced by dissolving the compound represented by General Formula (I) or a pharmaceutically acceptable salt thereof in a solvent, and then freeze-drying the solution.

Solvents used in the freeze-drying method include alcohols such as alcohols having 1 to 6 carbon atoms such as methanol, ethanol, 1-propanol, 2-propanol, and tert-butyl alcohol; ethers such as tetrahydrofuran; nitriles such as acetonitrile; and water, and these solvents can be used alone or as a mixed solvent of two or more types.

Though the particle size of the amorphous form of the compound represented by General Formula (I) or a pharmaceutically acceptable salt thereof is not particularly limited, examples of the volume average particle size (D50) include 20 µm or less, preferably include 1 to 15 µm, more preferably include 1 to 10 µm, still more preferably include 1.5 to 5 µm, and most preferably include 2 to 5 µm from the viewpoint of the effect of the invention and the production of preparations.

The volume average particle size (D50) can usually be measured by dispersing the measurement sample in a solvent such as water or ethanol and measuring the particle size distribution by a laser diffraction method. The measurement sample may be dispersed in a solvent by irradiation of ultrasonic waves or the like. The particle size distribution can be measured by a particle size distribution measuring device (for example, Shimadzu laser diffraction type particle size distribution measuring device SALD-2200). The volume average particle size (D50) can be determined from the obtained particle size distribution result. A commercially available software (for example, Shimadzu WingSALD-2200 version 1.02) can be used for data collection and analysis.

The "enteric-coated preparation" according to the present invention is a preparation designed to prevent decomposition of the active ingredient in the stomach or to release the active ingredient mainly in the small intestine without releasing it in the stomach. The enteric-coated preparation itself is listed in the Japanese Pharmacopoeia. As the enteric-coated preparation, dosage forms such as tablets, granules, fine granules, and capsules are known. Examples of the production method of these dosage forms include (i) a method in which enteric-coated granules in which an active ingredient or an active ingredient and a pharmaceutically acceptable additive are coated with an enteric polymer are produced, and tablets, granules, fine granules, or capsules containing the enteric-coated granules are produced, (ii) a method in which tablets, granules, fine granules, or capsules containing an active ingredient and a pharmaceutically acceptable additive are produced, and these preparations are coated with an enteric polymer, and (iii) a method in which an active ingredient or an active ingredient and a pharmaceutically acceptable additive are contained in a hard capsule composed of an enteric-coated base.

That is, examples of the enteric-coated preparation of the present invention include (i) tablets, granules, fine granules, or capsules containing enteric-coated granules in which an active ingredient or an active ingredient and a pharmaceutically acceptable additive are coated with an enteric polymer, (ii) tablets, granules, fine granules, or capsules containing an active ingredient and a pharmaceutically acceptable additive coated with an enteric polymer, and (iii) hard capsules in which an active ingredient or an active ingredient and a pharmaceutically acceptable additive are contained in a hard capsule composed of an enteric-coated base.

The enteric-coated base means a base composed of an enteric polymer known per se, and examples of the enteric polymer include enteric polymers exemplified as an enteric polymer for coating described later.

Examples of the enteric polymer for coating used in the present invention include enteric-coated methacrylic acid copolymers such as methacrylic acid copolymer L, methacrylic acid copolymer S (for example, Eudragit^{®} L100, Eudragit^{®} S100, manufactured by Evonik), methacrylic acid copolymer LD (for example, Eudragit^{®} L100-55, Eudragit^{®} L30D-55, manufactured by Evonik), methyl acrylate/methyl methacrylate/methacrylic acid copolymer (for example, Eudragit^{®} FS30D, manufactured by Evonik), enteric-coated cellulose polymers such as hypromellose (also referred to as hydroxypropylmethylcellulose), hypromellose acetate succinate (also referred to as hydroxypropylmethylcellulose acetate succinate) (manufactured by Shin-Etsu Chemical Co., Ltd., sometimes abbreviated as HPMCAS), hypromellose phthalate (also referred to as hydroxypropylmethylcellulose phthalate) (manufactured by Shin-Etsu Chemical Co., Ltd., sometimes abbreviated as HPMCP), carboxymethyl ethyl cellulose (manufactured by Freund Corp., sometimes abbreviated as CMEC), and cellacefate (also referred to as cellulose acetate phthalate), and enteric-coated vinyl alcohol polymers such as polyvinyl alcohol acetate phthalate (manufactured by Colorcon), and enteric-coated cellulose polymers and the like are preferable. Among these, hydroxypropylmethylcellulose, hydroxypropylmethylcellulose phthalate, and hydroxypropylmethylcellulose acetate succinate are preferable.

The enteric-coated granules can be produced according to a known method. For example, the enteric-coated granules can be produced by producing granules by, for example, fluidized bed granulation methods such as rolling fluidized bed granulation and fluidized bed granulation, rolling granulation methods such as centrifugal rolling granulation methods, or a stirring granulation methods or the like, and then coating the granules with an enteric coating liquid and drying them.

The enteric coating liquid can be prepared, for example, by a method of adding the enteric polymer to a solvent and concentrating the solvent, if necessary. Examples of the solvent used for preparing the enteric-coated liquid include water, alcohol solvents such as methanol and ethanol, and mixed liquids thereof. If necessary, pharmaceutically acceptable additives such as binding agents, plasticizers, coating bases, surfactants, and excipients can be appropriately compounded. Though the amount of the solvent is not particularly limited, the solvent can be used in an amount of 3 to 10 times the total weight of the substances to be dissolved (that is, the total weight of the enteric polymer and the pharmaceutically acceptable additive).

Tablets, granules, fine granules, or capsules containing an active ingredient and a pharmaceutically acceptable additive coated with the enteric polymer can be produced by producing tablets, granules, fine granules, or capsules containing an active ingredient and a pharmaceutically acceptable additive according to a known method, coating the obtained preparations with the enteric coating liquid, and drying them.

As the hard capsule composed of an enteric-coated base, a commercially available one can be used, for example, a hard capsule composed of an enteric-coated base containing hydroxypropylmethylcellulose or hydroxypropylmethylcellulose acetate succinate can be used, and more specifically, Vcaps^{®} Enteric (manufactured by Capsugel) and the like can be used.

The enteric-coated preparation of the present invention may contain a pharmaceutically acceptable additive as required, for example, a binding agent, a disintegrating agent, a excipient, a lubricant, and the like are appropriately combined and added in required amounts, and thereby a pharmaceutical composition of the present invention can be produced.

Examples of the binding agent include methyl cellulose, hydroxyethyl cellulose, hydroxypropyl cellulose, hypromellose, polyvinyl pyrrolidone, gelatin, agar, alginic acid, sodium alginate, partially saponified polyvinyl alcohol, pullulan, partly pregelatinized starch, dextrins, xanthan gum, and gum arabic powder. These may be used alone or two or more types thereof may be used in combination. Among these, hydroxypropyl cellulose, hypromellose, polyvinylpyrrolidone and the like are preferable.

Examples of the disintegrating agent include crystalline cellulose, carboxymethyl cellulose (also referred to as carmellose), croscarmellose sodium, calcium carboxymethyl cellulose, low-substituted hydroxypropyl cellulose, crospovidone, hydroxypropyl starch, starch, partly pregelatinized starch, and starch sodium glycolate. These may be used alone or two or more types thereof may be used in combination. Among these, croscarmellose sodium, sodium starch glycolate, crospovidone and the like are preferable, and crospovidone and the like are more preferable. An amount of the disintegrating agent added is preferably 5 to 30 weight% and more preferably 5 to 15 weight% based on a total weight of the particles containing the active ingredient. When it is added in tablets, the amount is preferably 1 to 10 weight%, more preferably 2 to 6 weight% based on a total weight of the tableting granules containing the active ingredient.

The excipient can be added in any of a kneading step, a granulation step, and a post-granulation end step of pharmaceutical preparations. Examples of the excipient include celluloses such as crystalline cellulose, ethyl cellulose, hydroxypropyl cellulose, low-substituted hydroxypropyl cellulose, and hydroxypropylmethylcellulose (also referred to as hypromellose), starches such as corn starch, potato starch, wheat starch, rice starch, partly pregelatinized starch, and hydroxypropyl starch, sugars such as glucose, lactose, white sugar, refined white sugar, powdered sugar, trehalose, dextran, and dextrins, sugar alcohols such as D-mannitol, xylitol, sorbitol, and erythritol, glycerin fatty acid esters, and inorganic salts such as magnesium aluminometasilicate, synthetic hydrotalcite, anhydrous calcium phosphate, precipitated calcium carbonate, calcium silicate, calcium hydrogen phosphate hydrate, and sodium hydrogen carbonate, and crystalline cellulose is preferable.

Examples of the lubricant include stearic acid, sodium stearyl fumarate, magnesium stearate, calcium stearate, sucrose fatty acid ester, polyethylene glycol, light anhydrous silicic acid, hardened oils, glycerin fatty acid esters, and a talc. These may be used alone or two or more types thereof may be used in combination. Among these, sodium stearyl fumarate, magnesium stearate, calcium stearate, sucrose fatty acid esters or the like is preferable.

In the enteric-coated preparation, by using the compound represented by General Formula (I) or a pharmaceutically acceptable salt thereof in combination with an enteric polymer, recrystallization of the compound represented by General Formula (I) or a pharmaceutically acceptable salt thereof from a supersaturated solution can be suppressed. When the compound represented by General Formula (I) or a pharmaceutically acceptable salt thereof and an enteric polymer are mixed, they are preferably uniformly mixed. The weight ratio of the compound represented by General Formula (I) or a pharmaceutically acceptable salt thereof to the enteric polymer is 1 : 0.5 to 1 :10, preferably 1 : 1 to 1 : 5, more preferably 1 : 2 to 1 : 5, and still more preferably 1 : 1 to 1 : 4.

Examples of the enteric polymer include the enteric polymer for coating, cellulose polymers and the like are preferable, and hydroxypropylmethylcellulose, hydroxypropylmethylcellulose phthalate, hydroxypropylmethylcellulose acetate succinate and the like are more preferable.

When the compound represented by General Formula (I) or a pharmaceutically acceptable salt thereof is mixed with the enteric polymer, the pharmaceutically acceptable additive can be appropriately added.

The enteric-coated preparation of the present invention can be orally or parenterally, more preferably orally administered to humans or other mammals, and is useful as a therapeutic agent for hyperuricemia or gout. The term "treatment" in this specification may include the concept of prophylactic or prophylactic use in addition to treatment.

One aspect of the present invention is a pharmaceutical composition containing enteric-coated granules in which the compound represented by General Formula (I) or a pharmaceutically acceptable salt thereof is coated with an enteric polymer.

Another aspect of the present invention is a tablet, a granule, a fine granule, or a capsule containing enteric-coated granules in which the compound represented by General Formula (I) or a pharmaceutically acceptable salt thereof is coated with an enteric polymer.

Another aspect of the present invention is a pharmaceutical composition that contains enteric-coated granules in which the compound represented by General Formula (I) or a pharmaceutically acceptable salt thereof is coated with an enteric polymer and in which the compound represented by General Formula (I) or pharmaceutically acceptable salt thereof in the enteric polymer is further mixed with an enteric polymer.

Another aspect of the present invention is a tablet, a granule, a fine granule, or a capsule that contains enteric-coated granules in which the compound represented by General Formula (I) or a pharmaceutically acceptable salt thereof is coated with an enteric polymer and in which the compound represented by General Formula (I) or a pharmaceutically acceptable salt thereof in the enteric polymer is further mixed with an enteric polymer.

Another aspect of the present invention is a pharmaceutical composition containing enteric-coated granules in which the compound represented by General Formula (I) or a pharmaceutically acceptable salt thereof and a pharmaceutically acceptable additive are coated with an enteric polymer.

Another aspect of the present invention is a tablet, a granule, a fine granule, or a capsule containing enteric-coated granules in which the compound represented by General Formula (I) or a pharmaceutically acceptable salt thereof and a pharmaceutically acceptable additive are coated with an enteric polymer.

Another aspect of the present invention is a pharmaceutical composition that contains enteric-coated granules in which the compound represented by General Formula (I) or a pharmaceutically acceptable salt thereof and a pharmaceutically acceptable additive are coated with an enteric polymer and in which the compound represented by General Formula (I) or a pharmaceutically acceptable salt thereof and the pharmaceutically acceptable additive in the enteric polymer are further mixed with an enteric polymer.

Another aspect of the present invention is a tablet, a granule, a fine granule, or a capsule that contains enteric-coated granules in which the compound represented by General Formula (I) or a pharmaceutically acceptable salt thereof and a pharmaceutically acceptable additive are coated with an enteric polymer and in which the compound represented by General Formula (I) or a pharmaceutically acceptable salt thereof and the pharmaceutically acceptable additive in the enteric polymer are further mixed with an enteric polymer.

Another aspect of the present invention is a pharmaceutical composition containing the compound represented by General Formula (I) or a pharmaceutically acceptable salt thereof and a pharmaceutically acceptable additive coated with an enteric polymer.

Another aspect of the present invention is a tablet, a granule, a fine granule, or a capsule containing the compound represented by General Formula (I) or a pharmaceutically acceptable salt thereof and a pharmaceutically acceptable additive coated with an enteric polymer.

Another aspect of the present invention is a pharmaceutical composition that contains the compound represented by General Formula (I) or a pharmaceutically acceptable salt thereof and a pharmaceutically acceptable additive coated with an enteric polymer and in which the compound represented by General Formula (I) or a pharmaceutically acceptable salt thereof and the pharmaceutically acceptable additive are further mixed with an enteric polymer.

Another aspect of the present invention is a tablet, a granule, a fine granule, or a capsule that contains the compound represented by General Formula (I) or a pharmaceutically acceptable salt thereof and a pharmaceutically acceptable additive coated with an enteric polymer and in which the compound represented by General Formula (I) or a pharmaceutically acceptable salt thereof and the pharmaceutically acceptable additive are further mixed with an enteric polymer.

Another aspect of the present invention is a hard capsule in which the compound represented by General Formula (I) or a pharmaceutically acceptable salt thereof is contained in a hard capsule composed of an enteric-coated base.

Another aspect of the present invention is a hard capsule in which the compound represented by General Formula (I) or a pharmaceutically acceptable salt thereof and a pharmaceutically acceptable additive are contained in a hard capsule composed of an enteric-coated base.

Another aspect of the present invention is a hard capsule in which the compound represented by General Formula (I) or a pharmaceutically acceptable salt thereof is contained in a hard capsule composed of an enteric-coated base and the compound represented by General Formula (I) or a pharmaceutically acceptable salt thereof is further mixed with an enteric polymer.

Another aspect of the present invention is a hard capsule in which the compound represented by General Formula (I) or a pharmaceutically acceptable salt thereof and a pharmaceutically acceptable additive are contained in a hard capsule composed of an enteric-coated base and the compound represented by General Formula (I) or a pharmaceutically acceptable salt thereof and the pharmaceutically acceptable additive are further mixed with an enteric polymer.

Another aspect of the present invention is a method of treating hyperuricemia or gout including administering a therapeutically effective amount of the enteric-coated preparation of the present invention to a subject for which a hyperuricemia or gout treatment is necessary. The dose of the enteric-coated preparation of the present invention can be adjusted according to the content of the compound represented by General Formula (I) or a pharmaceutically acceptable salt thereof in the enteric-coated preparation of the present invention. In addition, the dose can be appropriately determined according to an administering method, and age, body weight, gender, symptoms and sensitivity to a drug of an administering subject, and the like, but it may be adjusted according to the progress of improvement in symptoms.

A dose of the enteric-coated preparation of the present invention, for example, in terms of a content of the compound represented by General Formula (I) or a pharmaceutically acceptable salt thereof for adults, of 1 mg to 2000 mg per day for oral administration can be usually administered, but it can be increased or decreased according to age, symptoms, and the like. In addition, the number of administrations is, for example, 1 to 3 times per day, and preferably 1 to 2 times.

### EXAMPLES

The present invention will be described below in further detail with reference to examples, comparative examples, and test examples, but the present invention is not limited thereto.

### <Example 1 and Comparative Example 1, production of enteric-coated capsules>

After dissolving 9 g of Compound 14 (2-(3-cyano-4-phenoxyphenyl)-7-hydroxythiazolo[5,4-d]pyrimidine) in 1936 g of a mixed solvent of tetrahydrofuran (may be abbreviated as THF), ethanol, and water (weight ratio:
THF/ethanol/water = 1600.5/254.5/81) (dissolved by slightly heating), the solution was pumped into a spray dryer at a rate of about 5 mL/min via a peristaltic pump and spray dried and granulated from a 2-fluid nozzle (with a diameter of 508 µm) at an inlet temperature of 80°C and an outlet temperature of about 60°C under conditions of a dry air flow of 0.30 m³/min and a nozzle spray air pressure of 1.0 kgf/cm². The obtained dried component was allowed to stand overnight at room temperature to obtain 100% amorphous Compound 14.

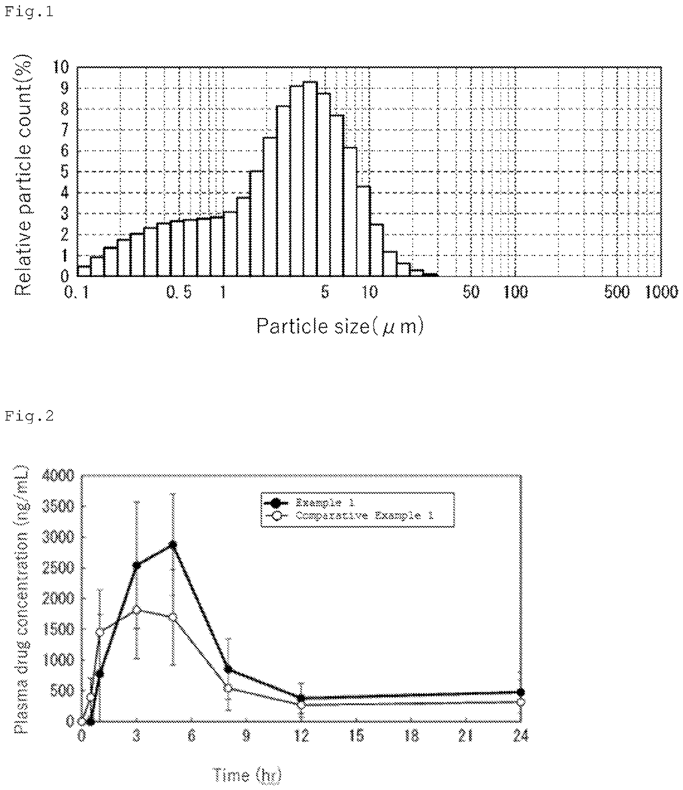

A volume average particle size (D50) of the obtained amorphous Compound 14 was measured with a Shimadzu laser diffraction type particle size distribution measuring device SALD-2200 using a dispersion obtained by adding about 2 mg of a sample to a 0.2% Aerosol OT aqueous solution and irradiating the solution with ultrasonic waves for 30 seconds for dispersion. Shimadzu WingSALD-2200 version 1.02 software was used for data collection and analysis. Fig. 1 shows the result of the particle size distribution of amorphous Compound 14. The volume average particle size (D50) of amorphous Compound 14 obtained from the particle size distribution was 2.949 µm.

Each of the capsules shown in Table 2 was filled with 40 mg of the obtained amorphous Compound 14 to obtain capsules of amorphous Compound 14.

**[Table 2]**

| | Capsule name | Enteric-coated | Component of capsule | Manufacturer of capsule |
|---|---|---|---|---|
| Example 1 | Vcaps^{®} Enteric | Yes | HPMCAS/HPMC | Lonza/Capsugel |
| Comparative Example 1 | Vcaps Plis^{®} | No | HPMC | Lonza/Capsugel |

### <Test Example 1, Absorbability of amorphous Compound 14 in dogs>

Single oral administration of the capsules of Example 1 and Comparative Example 1 (40 mg/body as Compound 14) was performed on beagle dogs (1 to 5 years old, KITAYAMA LABES CO., LTD.) that had been fasted from the evening of the day before the administration one capsule at a time by a crossover method with a one-week administration interval. Blood was collected from the cephalic vein, and the time of the collection was 0.5, 1, 3, 5, 8, and 24 hours before and after the administration. The obtained blood was centrifuged at 10000 ×g and 4°C for 5 minutes to obtain plasma. The concentration of Compound 14 in the plasma was measured by HPLC (OSAKA SODA CO., LTD.). Since dogs generally have a high gastric pH, pentagastrin, a gastric acid stimulator, was intramuscularly injected at a dose of 0.01 mg/kg 30 minutes before and immediately before the administration of the capsules. Then, the pH of a gastric solution was measured to confirm that the gastric pH was low, and the capsule sample was administered.

Plasma drug concentration-time curves were created from the obtained measurement values. The results are shown in Fig. 2. The drug concentrations in the figure show the mean value ± standard deviation of four examples.

When the enteric-coated capsule of Example 1 was used, the drug concentration was higher than that when the general gastric-soluble capsule of Comparative Example 1, which was not enteric-coated, was used. Thus, absorbability was shown to be improved by the enteric-coating of amorphous Compound 14.

### <Test Example 2, Evaluation of amorphous state of Compound 14 (powder X-ray diffraction)>

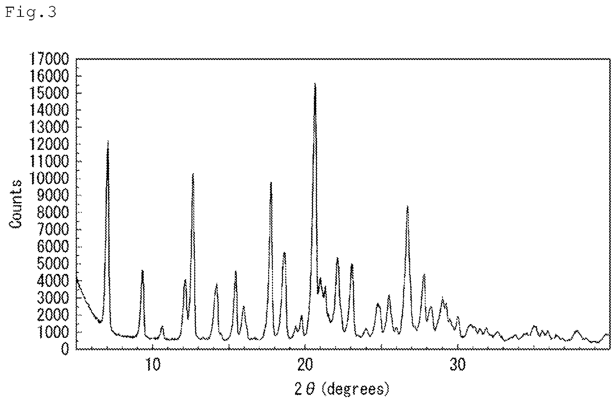

Since it is very important that an amorphous substance retains amorphous properties even after storage over time, amorphous Compound 14 of Example 1 was stored at room temperature in a light-shielded and airtight manner, and a change in the amorphous state of Compound 14 was evaluated using an X-ray diffraction device (D2 Phaser, Bruker). The results are shown in Figs. 4(a) to (d). For comparison, Fig. 3 shows the crystalline powder X-ray diffraction of Compound 14.

When the capsule of Example 1 was stored at room temperature in a light-shielded and airtight manner, changes in the powder X-ray diffraction pattern of Compound 14 over time were not observed during the storage for 4 weeks.

### INDUSTRIAL APPLICABILITY

The enteric-coated preparation of the present invention is useful as a therapeutic agent for hyperuricemia and the like because it exhibits excellent in vivo absorbability of an active ingredient and storage stability.

## Claims

1. An enteric-coated preparation, comprising:
a compound represented by General Formula (I) 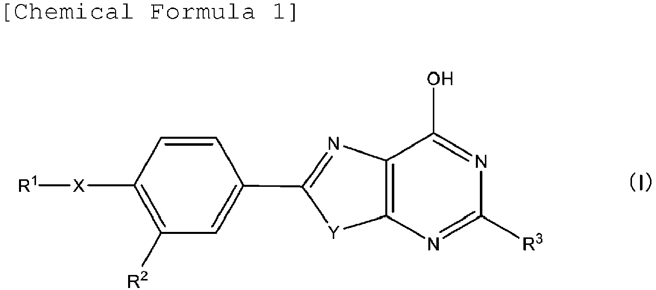
wherein R¹ is an unsubstituted phenyl group or a phenyl group substituted with a substituent, the substituent being at least one group selected from the group consisting of an alkyl group having 1 to 8 carbon atoms, an alkyl group having 1 to 8 carbon atoms substituted with a halogen atom, an alkoxy group having 1 to 8 carbon atoms, an alkoxycarbonyl group having 2 to 8 carbon atoms, a formyl group, a carboxyl group, a halogen atom, a phenyl group and a phenoxy group, R² is a cyano group or a nitro group, R³ is a hydrogen atom or a hydroxyl group, X is an oxygen atom or -S(O)ₙ-, n is an integer of 0 to 2, and Y is an oxygen atom or a sulfur atom,
or a pharmaceutically acceptable salt thereof.

2. The enteric-coated preparation according to Claim 1, wherein R¹ is an unsubstituted phenyl group or a phenyl group substituted with a halogen atom.

3. The enteric-coated preparation according to Claim 1 or 2, wherein X is an oxygen atom.

4. The enteric-coated preparation according to any one of Claims 1 to 3, wherein Y is a sulfur atom.

5. The enteric-coated preparation according to any one of Claims 1 to 4, wherein the compoundor a pharmaceutically acceptable salt thereof according to any one of Claims 1 to 4 includes an amorphous form thereof, and a content of the amorphous form is 80 weight% or more based on a total weight of the compound or a pharmaceutically acceptable salt thereof according to any one of Claims 1 to 4.

6. The enteric-coated preparation according to any one of Claims 1 to 5, which is a hard capsule.
